# EUROPEAN PATENT APPLICATION

(11) **EP 1 293 204 A1**
(43) Date of publication of application: **19.03.2003**
(21) Application number: 01925954.8
(22) Date of filing: 26.04.2001
(51) Int. Cl.: A61K 31/17, A61K 9/06, A61K 47/32, A61K 47/36, A61K 7/00, A61K 7/48, A61P 17/00

(54) **UREA-CONTAINING GEL PREPARATION**

(30) Priority: 26.04.2000 JP 2000125344
(71) Applicant: Kowa Co., Ltd., Naka-ku, Nagoya-shi, Aichi 460-0003 (JP)
(72) Inventor: SAWADA, Yoshitoshi, FUJI-SHI, Shizuoka 417-0855 (JP); YAMAMOTO, Shinya, FUJI-SHI, Shizuoka 416-0944 (JP); SUEHIRO, Saibi, NUMAZU-SHI, Shizuoka 410-0306 (JP); INAGI, Toshio, MISHIMA-SHI, Shizuoka 411-0038 (JP); SHINODA, Yasuo, SHIZUOKA-SHI, Shizuoka 420-0867 (JP)
(74) Representative: Wächtershäuser, Günter, Prof. Dr.
(86) International application number: JP0103622
(87) International publication number: WO01080850

(57) **Abstract**

A gel preparation comprising urea, an acrylic acid polymer and a xanthan gum.

The gel preparation does not change much in viscosity with temperature, contains the urea stably, has high moisture-retaining effect for the skin, does not give much feeling of tackiness, and is excellent in feeling of use.

## Description

### Technical Field

This invention relates to a urea-containing gel preparation, which contains urea having pharmacological effects such as moisture-retaining effect, protein melting/unfolding action and antimicrobial activities and being effective for the treatment of xeroderma, keratosis, ichthyosis and the like, does not change much in viscosity with time at varied temperatures, has high moisture-retaining effect for the skin, does not give much feeling of tackiness, and is excellent in feeling of use.

### Background Art

External preparations with urea added therein for the treatment of xeroderma, keratosis, ichthyosis and the like are known for years, and creams, ointments and lotion preparations with urea added at 10 or 20 wt.% have been put on the market. Many of these commercial products are emulsion preparations which make use of emulsifiers.

A water-soluble gel preparation, on the other hand, is considered to retain skin-moisturizing effect over a long time, because it forms a high molecular film on a skin surface, increases the transdermal absorption of an active ingredient and further, prevents evaporation of water from the skin. In view of this, there is a long standing desire for the commercialization of water-soluble gel preparations with urea added therein.

Known as gel preparations with urea added therein include, for example, those disclosed in JP-B-62-041645 and JP-B-06-021061.

Each preparation disclosed in JP-B-62-041645 contains an ammonium compound added therein to suppress decomposition of urea. It is, however, accompanied by a problem that synergistic action of the ammonium compound and ammonia occurring through decomposition of urea with time intensifies an ammonia smell and lowers its commercial value.

Each preparation disclosed in JP-B-06-021061, on the other hand, contains a great deal of a polyhydric alcohol as a base, so that due to the polyhydric alcohol of high concentration and a high molecular compound, the preparation itself is hard and has pronounced post-application tackiness which is specific to gel preparations. Moreover, the preparation undergoes substantial changes in consistency with time, and is by no means suited for use.

In addition, a water-soluble gel preparation with urea added therein generally has a high water content so that its consistency changes considerably with temperature when the content of a gelling agent is low. This preparation, therefore, has a strong tendency that its physical properties undergo substantial changes as typified, for example, by liquefaction or separation of a water layer. An increase in the content of the gelling agent in an attempt to avoid variations in consistency, however, results in a very hard preparation which gives intensified post-application feeling of tackiness and extremely deteriorated feeling of use.

As has been described above, it is the current circumstance that no water-soluble gel preparation with urea added therein has been developed and marketed as a commercial product yet to date for the difficulty in its formulation due to drawbacks such as intensification of its ammonia smell with time, poor feeling of use such as feeling of tackiness and significant changes in consistency with time at varied temperatures.

An object of the present invention is, therefore, to provide a urea-containing gel preparation, which is reduced in consistency changes with time at varied temperatures, has high moisture-retaining effect for the skin, does not give much feeling of tackiness, and is excellent in feeling of use.

### Disclosure of the Invention

The present inventors have proceeded with extensive research to achieve the above-described object. As a result, it has been found that addition of two types of gelling agents, an acrylic acid polymer and a xanthan gum, along with urea makes it possible to obtain a gel preparation reduced in consistency changes with time at varied temperatures and containing the urea stably and also that further addition of a silicone oil makes it possible to obtain a urea-containing gel preparation which has still higher water-retaining effect for the skin, does not give much feeling of tackiness and is excellent in feeling of use, leading to the completion of the present invention.

Described specifically, the present invention provides a gel preparation comprising urea, an acrylic acid polymer and a xanthan gum.

The present invention also provides a gel preparation comprising urea, an acrylic acid polymer, a xanthan gum and a silicone oil.

### Brief Description of the Drawings

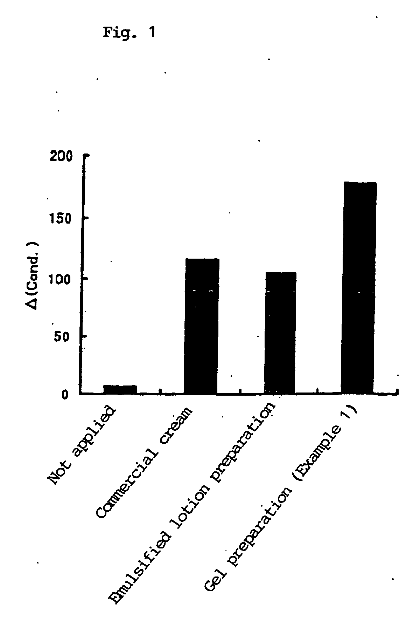

FIG. 1 is a histogram illustrating the contents of water in the skin upon elapsed time of 4 hours after application of preparations in Test 2.

### Best Modes for Carrying Out the Invention

The content of urea for use in the present invention may range preferably from 1 to 30 wt.%, more preferably from 5 to 25 wt.%, most preferably from 10 to 20 wt.%.

No particular limitation is imposed on the acrylic acid polymer for use in the present invention, insofar as it is a polymer composed principally of a polyacrylic acid and containing carboxyl groups in its molecule. Preferred examples are carboxyvinyl polymers although those obtained by mixing and copolymerizing, for example, other carboxylic acid monomers, such as methacrylic acid, chloroacrylic acid, cyanoacrylic acid and maleic anhydride, or alkyl ethers of polyhydric alcohols in small proportions are also included. As carboxyvinyl polymers, commercially-available, powdery, water-soluble acrylic acid polymers can be used. Illustrative are "Carbopol", "Noveon" and "Pemulen" (products of BF Goodrich Chemical Co.); "HIVISWAKO" (products of Wako Pure Chemical Industries, Ltd.); and "Synthalene" (products of SV Sigma SpA). Specific examples can include "Carbopol 934", "Carbopol 934P", "Carbopol 940", "Carbopol 941", "Carbopol 971P", "Carbopol 974P", "Carbopol 980", "Carbopol 981", "Carbopol 910", "Carbopol 1342", "Carbopol 2984", "Carbopol 5984", "Carbopol EX214", "Carbopol ULTRE210" and "Carbopol ETD2050"; "Noveon AA-1"; "Pemulen TR-1" and "Pemulen TR-2"; "HIVISWAKO 103", "HIVISWAKO 104", "HIVISWAKO 105", "HIVISWAKO 204" and "HIVISWAKO 304"; and "Synthalene K", "Synthalene L" and "Synthalene M".

The content of the acrylic acid polymer may preferably be from 0.05 to 3 wt.%, with 0.1 to 1.5 wt. % being particularly preferred.

The xanthan gum for use in the present invention is a microorganism-derived natural gum which is obtained by fermenting glucose with Xanthomonas campestris, and illustrative are acidic polysaccharides including D-glucose, D-mannose and D-glucuronic acid.

The content of the xanthan gum may preferably be from 0.05 to 3 wt.%, with 0.1 to 1.5 wt.% being particularly preferred.

According to the present invention, the addition of the two kinds of high molecular compounds, that is, the acrylic acid polymer and the xanthan gum has made it possible to stabilize the gel preparation. The content ratio (weight ratio) of the acrylic acid polymer to the xanthan gum may preferably be from 0.05:3 to 3:0.05, with 0.1:1.5 to 1.5:0.1 being particularly preferred.

To the gel preparation according to the present invention, a water-soluble, high molecular compound other than an acrylic acid polymer or a xanthan gum can also be added further. Examples of such other water-soluble, high molecular compound can include cellulose-based, high molecular compounds such as carboxymethylcellulose and salts thereof, methylcellulose, hydroxypropyl cellulose, and hydroxypropyl methylcellulose; vinyl-containing, high molecular compounds such as polyvinyl alcohol and polyvinyl pyrrolidone; and polysaccharides such as alginic acid and salts thereof.

To the gel preparation according to the present invention, a silicone oil can also be added further. Its addition is preferred because still better feeling of use is available. Examples of such a silicone oil can include dimethylpolysiloxane and methylpolysiloxane. Its content may preferably be from 0.01 to 5 wt.%, with 0.1 to 2 wt.% being particularly preferred.

The gel preparation according to the present invention can also contain, in addition to the above-described ingredients, one or more oil ingredients, for example, hydrocarbons such as squalane and liquid paraffin and esters such as isopropyl myristate, diisopropyl adipate and octyldodecyl myristate.

Their total content may preferably be from 0.01 to 5 wt.%, with 0.1 to 2 wt.% being particularly preferred.

It is also possible to add, as a stabilizer for urea, a neutral amino acid such as aminoacetic acid or DL-alanine; as a chelating agent, sodium edetate; as a preservative, parabene or the like; and a buffer such as citrate buffer or phosphate buffer.

Moreover, one or more of humectants and other active ingredients may also be added as needed. Any humectants can be used insofar as it is commonly employed in external preparations or cosmetic preparations. Illustrative are polyhydric alcohols such as glycerin, propylene glycol and 1,3-butylene glycol, lactic acid, and sodium lactate. Such a humectant can also be used as a water-soluble base, and may be added preferably in a proportion of 10 wt.% or less based on the whole composition.

Examples of the other active ingredients can include anti-inflammatories such as the potassium and ammonium salts of glycyrrhizic acid; and antihistamines useful for the treatment of itch, such as diphenhydramine hydrochloride.

To the gel preparation according to the present invention, an inorganic or organic basic substance can also be added for adjusting its pH. Illustrative are inorganic bases such as sodium hydroxide and potassium hydroxide; and organic bases such as aqueous ammonia, diethylamine and triethanolamine.

It is preferred to add the inorganic or organic base such that the pH of the gel preparation falls within a range of from 5 to 9, especially from 6 to 8.5.

Further, the content of water may preferably be from 40 to 90 wt.%, with 55 to 85 wt.% being particularly preferred, although it is adjusted depending upon the contents of other ingredients.

No particular limitation is imposed on the process for the production of the gel preparation according to the present invention. In general, however, it can be produced by adding high molecular compounds such as an acrylic acid polymer and a xanthan gum to water and allowing them to swell, adding and dissolving urea, adding to the resulting solution bases such as a polyhydric alcohol, citric acid and aminoacetic acid and thoroughly stirring and mixing the thus-prepared mixture, and then adding an alkaline, neutralizing agent such as an aqueous solution of sodium hydroxide and thoroughly stirring and mixing to adjust the pH of the preparation to 5 to 9. An oil base such as a silicone oil can also be dispersed further as needed.

From the standpoint of ease in filling in containers and feeling of use, the viscosity at 25°C of the gel preparation according to the present invention may preferably be from 20,000 to 70,000 cP in general, with 25,000 to 50, 000 cP being particularly preferred, as measured by the method described in the Pharmacopoeia of Japan.

Incidentally, the term "consistency" as used herein is an index which indicates the spreadability of a gel preparation, an ointment or the like. Its value differs depending upon the temperature, container, measuring instrument, measuring conditions and the like. When measured, for example, by the method shown in Test 1 to be described subsequently herein, the consistency at 25°C may preferably be a value of from 2 to 15 g, with a value of from 3 to 10 g being particularly preferred. A consistency value smaller than 2 g results in a gel which liquefies and runs off from a container or an applied part, while a consistency value greater than 15 g leads to a gel which is so hard that it cannot be easily squeezed out of a container or cannot be easily caused to spread upon application.

### Examples

The present invention will hereinafter be described in further detail based on Examples. It should, however, be borne in mind that the present invention is not limited to the following Examples.

### Example 1

A carboxyvinyl polymer ("Carbopol ETD2050", 0.9 g) and sodium edetate (0.01 g) were added to purified water (35 g), followed by thorough stirring and mixing. To the resulting solution, a dispersion of xanthan gum (0.3 g) in concentrated glycerin (2.8 g) was added. Subsequent to thorough stirring and mixing, the thus-obtained mixture was allowed to stand overnight under reduced pressure (about 50 kg/cm²) (Prepared Liquid A). Subsequently, aminoacetic acid (3 g), methylparabene (0.05 g) and citric acid (0.3 g) were added to purified water (33.45 g), and the resulting mixture was heated under stirring and mixing. When its temperature arose to about 60°C, urea (20 g) was added, and under stirring and mixing, was allowed to dissolve (Prepared Liquid B). Prepared Liquid B was added to Prepared Liquid A. Subsequent to thorough stirring and mixing at about 35°C under reduced pressure (about 50 kg/cm²), a solution of sodium hydroxide (0.49 g) in purified water (2 g) was added, followed by thorough stirring and mixing at about 30°C or lower under reduced pressure (about 50 kg/cm²). A dispersion of dimethylpolysiloxane (0.5 g) in concentrated glycerin (1.2 g) was added further, followed by thorough stirring and mixing under reduced pressure (about 50 kg/cm²) to produce a gel preparation.

By a similar procedure, gel preparations of Examples 2-6 and Comparative Examples 1-6, compositions of which are shown in Table 1 and Table 2, were alsoproduced. Incidentally, methylcellulose, hydroxypropyl cellulose or sodium alginate was added along with the carboxyvinyl polymer.

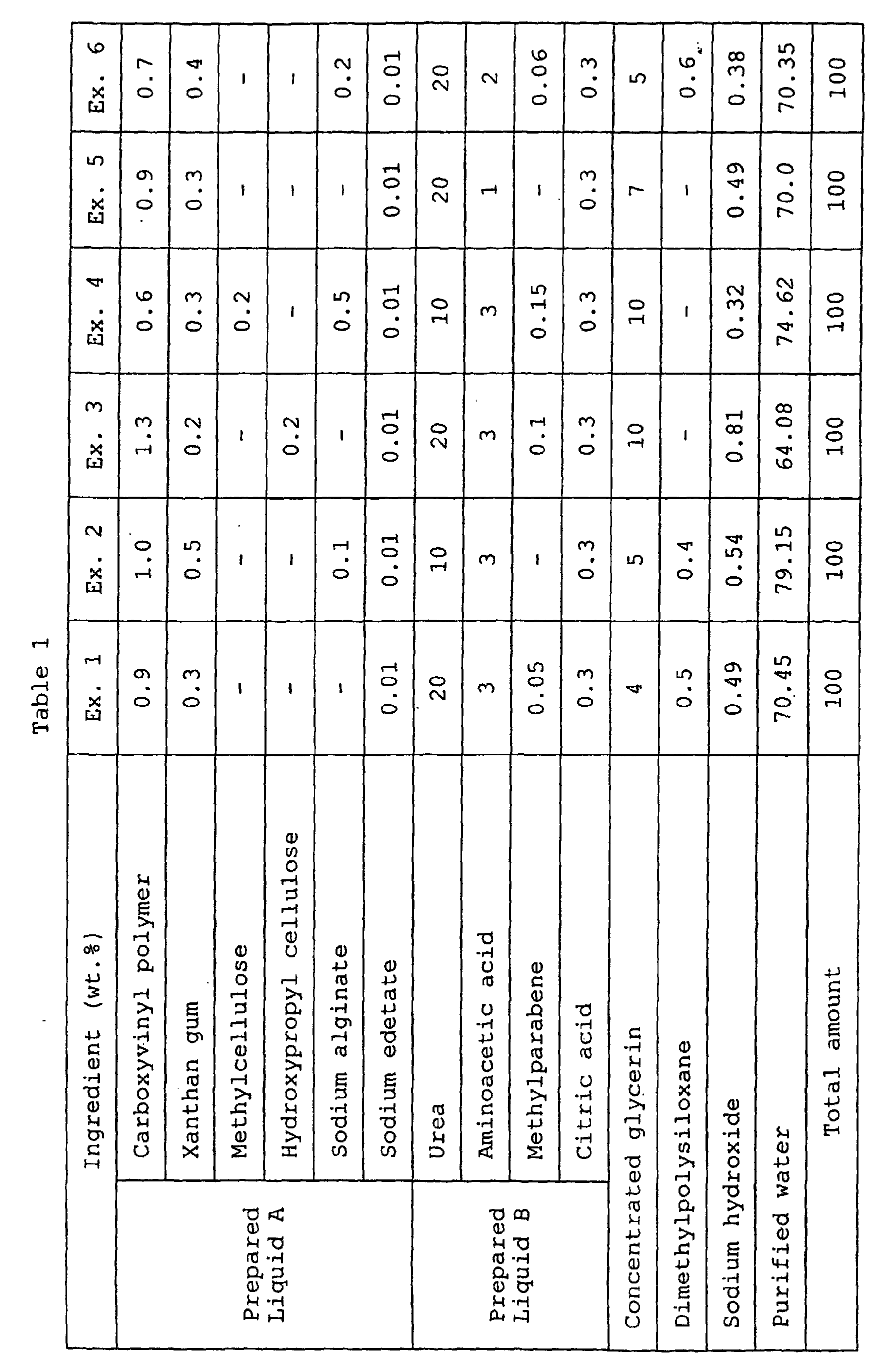

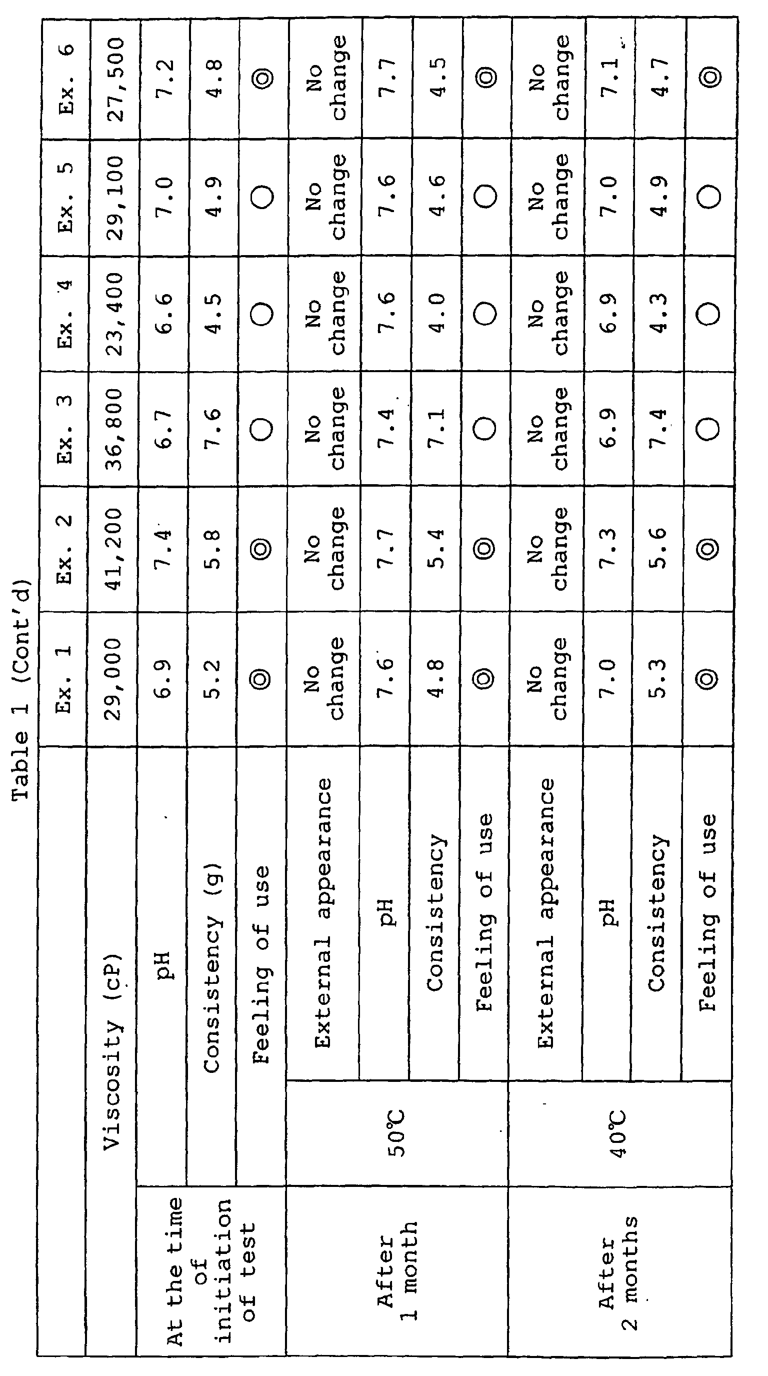

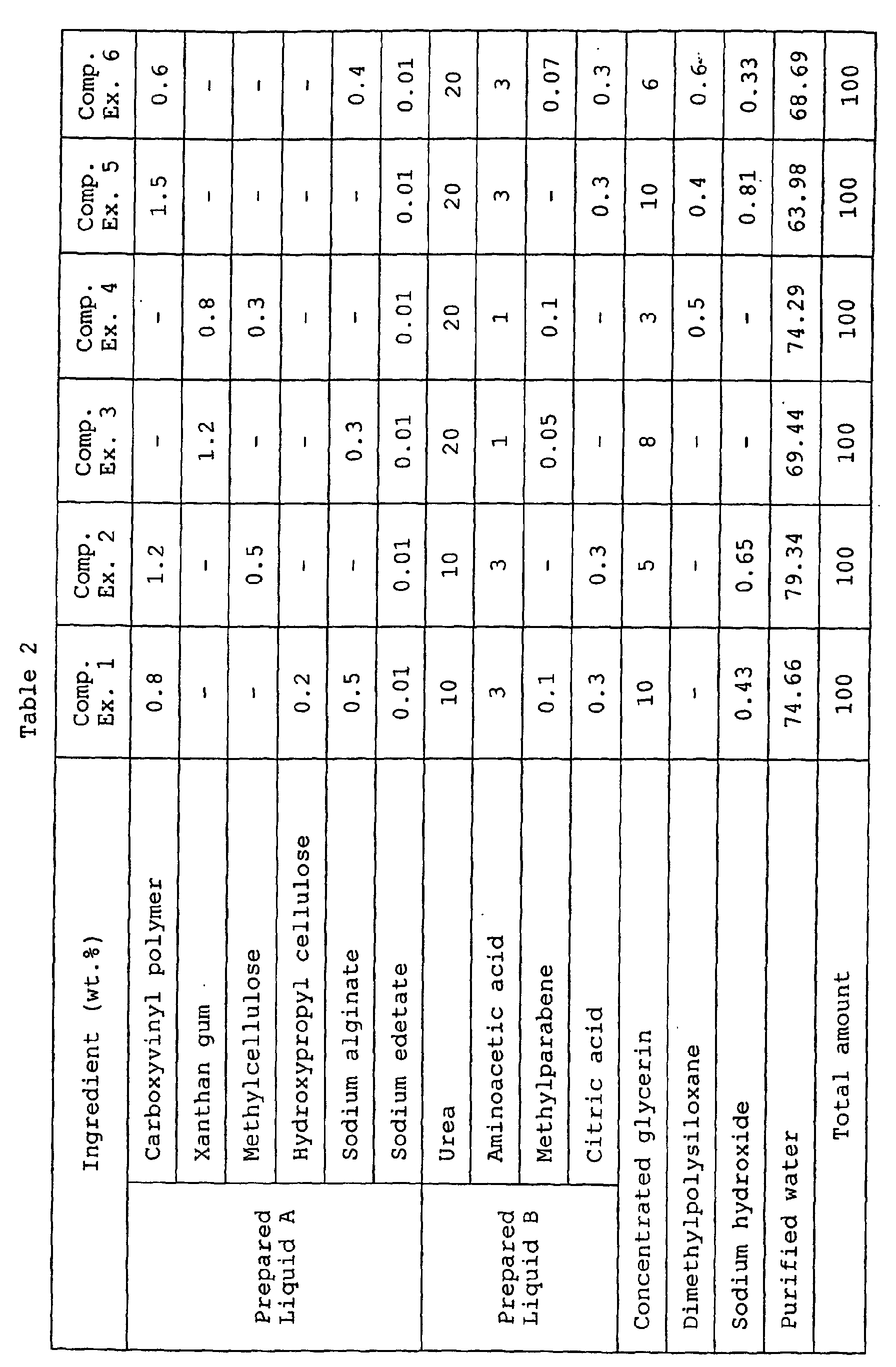

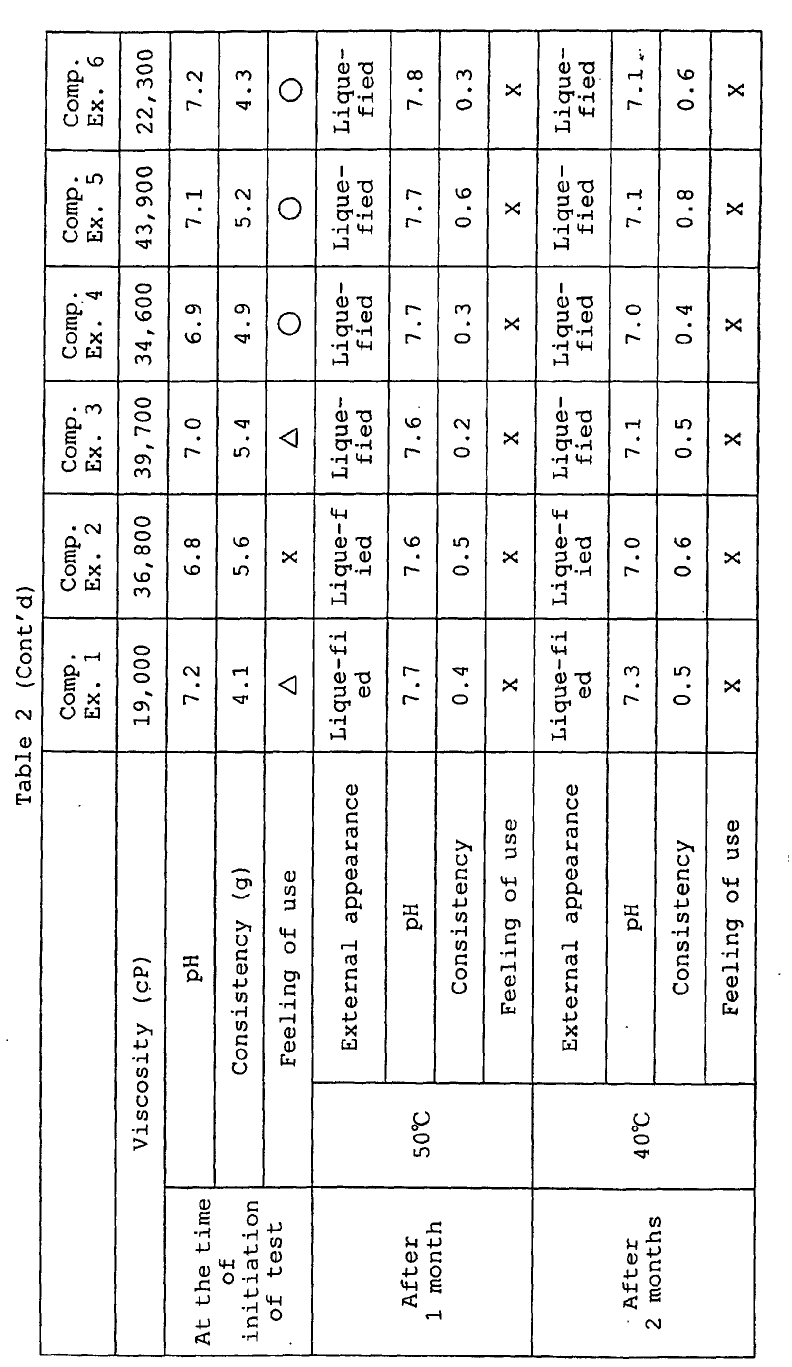

### Test 1

The gel preparations of Examples 1-6 and Comparative Examples 1-6 were measured for viscosity, consistency and pH and also ranked in terms of external appearance and feeling of use (at the time of initiation of the test, after stored at 50°C for 1 month and after stored at 40°C for 2 months). The results are shown in Table 1 and Table 2.

Incidentally, the viscosity (cP) of each preparation was calculated by multiplying with a constant a value obtained when, after the preparation filled in a container had been allowed to stand overnight in a constant-temperature chamber controlled at 25°C, the preparation was placed in a BL type rotational viscometer equipped with a No. 4 rotor (manufactured by Tokimec Inc.) and the rotor was then driven for 30 seconds. That procedure was repeated three times, and an average of the viscosity values so obtained was recorded as a viscosity (cP) at 25°C.

Employed as the consistency of each preparation was a value (g) obtained by allowing the preparation filled in a 10K bottle to stand overnight in a constant-temperature chamber controlled at 25°C and then measuring its consistency by a rheometer (manufactured by Fudo Kogyo Co., Ltd.) under the following conditions - adapter: ball of 10 mm in diameter, travel distance: 10 mm, speed: 5 cm/min.

As the pH of each preparation, a measurement was conducted on a solution formed by adding water (9 mL) to the preparation (1 g) and stirring andmixing the resultant mixture. The external appearance of each preparation was visually observed.

Concerning the feeling of use, each gel preparation was applied to the skin of a normal volunteer, and its feeling of use was organoleptically ranked in accordance with the following 4-level grading scale:
X: Tackiness is felt too strong to permit actual use.
Δ: Tackiness is felt, but the preparation may be used depending upon where it is applied.
○: Some tackiness is felt depending upon the amount applied.
ⓞ: Tack-free feeling is obtained, and good feeling of application is available.

From the results of Table 1 and Table 2, the preparations of Examples 1-6, to each of which the carboxylvinyl polymer and xanthan gum were added, underwent only small changes in consistency, and separation, discoloration and the like were not observed thereon. As feeling of use, they were smooth and showed good spreadability. Especially, the preparations of Examples 1, 2 and 6 were felt tack-free when applied, and gave good feeling of use.

In contrast, the preparations of Comparative Examples 1-6, after stored at 50°C for 1 month or at 40°C for 2 months, were all lowered in consistency so that they were no longer able to retain a gel structure. When the preparations were applied, they hence ran off from the skin, andmoreover, tackiness was felt strong and feeling of use was extremely poor.

### Test 2

With respect to a gel preparation according to the present invention (Example 1), a commercially-available cream preparation containing 20% of urea and the emulsified lotion preparation of Example 2 of JP-A-09-249555, all of which contained 20% of urea, skin water contents were measured upon an elapsed time of 4 hours after the application.

Described specifically, hair was removed from the dorsal skin of each rat (Wistar strain, male, 8-weeks old) by hair clippers and a depilatory, and a sample (0.1 g) was applied onto an area of 2 x 2 cm (4 cm²). Four hours later, the sample was wiped off with absorbent wadding soaked with ethanol, and then, the content of water in the skin was measured by a stratum corneum water content meter (manufactured by I.B.S. Company) in accordance with the high-frequency conductance measuring method. The results are shown in FIG 1.

From the results of FIG. 1, the gel preparation according to the present invention is evidently superior in water retaining property than the commercially available cream preparation or emulsified lotion preparation.

### Industrial Applicability

The gel preparation according to the present invention does not undergo much changes in consistency at varied temperatures, contains urea stably, has high water-retaining effect for the skin, does not give much tacky feeling, and is excellent in feeling of use.

## Claims

1. A gel preparation comprising urea, an acrylic acid polymer and a xanthan gum.

2. A gel preparation comprising urea, an acrylic acid polymer, a xanthan gum and a silicone oil.

3. A gel preparation according to claim 1 or 2, wherein said acrylic acid polymer is a carboxyvinyl polymer.

4. A gel preparation according to any one of claims 1-3, wherein a content ratio (weight ratio) of said acrylic acid polymer to said xanthan gum is from 0.05:3 to 3:0.05.

5. A gel preparation according to any one of claims 1-4, a consistency at 25°C of which is from 2 to 15 g.

6. A gel preparation according to any one of claims 1-5, a viscosity at 25°C of which is from 20,000 to 70,000 cP.
